# EUROPEAN PATENT APPLICATION

(11) **EP 4 155 705 A1**
(43) Date of publication of application: **29.03.2023**
(21) Application number: 21198435.6
(22) Date of filing: 23.09.2021
(51) Int. Cl.: G01L 9/00, A61B 5/0215, B81B 3/00, B81B 7/00

(54) **PRESSURE SENSOR**

(71) Applicant: BIOTRONIK SE & Co. KG, 12359 Berlin (DE)
(72) Inventor: SKERL, Olaf, 18209 Bad Doberan (DE); VAN OOYEN, André, 10717 Berlin (DE); ROMBERG, Jan, 12347 Berlin (DE)
(74) Representative: Biotronik Corporate Services SE

(57) **Abstract**

The present invention relates to a pressure sensor for pressure measurement in an aggressive medium comprising at least two stationary electrodes. The pressure sensor further comprises a movable element adapted to move at least in part based on an external pressure, wherein the movable element is arranged in a vicinity of the at least two electrodes such that an electric field generated by the at least two electrodes changes at least in part based on a movement of the movable element.

## Description

The present invention relates to a pressure sensor for pressure measurement in an aggressive medium.

Pressure sensors for use in a medium generally include a movable element, such as a membrane. It must be adapted for determining a pressure difference between an inner side of the movable element and an outer side of the movable element which stays in contact with the medium of interest. As this concept has seen continued research and development, issues regarding precise pressure measurements have come to light. Since in sensors comprising a membrane or diaphragm the pressure is generally determined based on a bending of the membrane caused by the surrounding medium, particularly the information transfer of the degree of bending to an electrical evaluation circuit and/or a transducer is critical.

There have been attempts in the prior art to arrange the evaluating circuit/transducer inside a cavity filled with a coupling liquid. In this manner, the membrane basically prevents a mixing of the coupling liquid and the surrounding medium, while the external pressure applied to the membrane is directly transferred over the coupling liquid onto a transducer inside the cavity. However, thermal expansion and aging effects of the coupling liquid negatively influence precise pressure measurements. Furthermore, already small bubbles inside the coupling liquid introduced during a filling process incorporate measurement errors, which are not easily compensated by any evaluation routine.

Alternatively, instead of using a coupling liquid inside the pressure sensor, attempts have been made to attach strain transducers, such as piezoresistive materials or similar, directly onto the membrane to receive information about a degree of membrane bending which can be utilized for determining the external pressure. However, creeping of the fixing material or adhesive required for attaching the strain transducer on the membrane mostly leads to a high drift over time for such pressure sensors. Furthermore, attaching a plurality of strain transducers on the membrane not only requires a complex and costly manufacturing process, but also massively influences the behavior of the membrane, leading to additional uncertainties for the pressure value of interest.

A further alternative known in the art is to determine the pressure based on the functional principal of a capacitor in which the membrane corresponds to one capacitor plate. In this manner, a pressure induced movement of the membrane leads to a change in an electric signal, e.g., a capacitance or a voltage, which can be used for deriving the external pressure. However, using the membrane as capacitor electrode increases the sensitivity for noise and additional measurement uncertainties, since the membrane and the evaluation circuitry are in electrical contact. Furthermore, since the membrane is generally in contact with the medium surrounding the sensor, any fluctuations of the (electric) potential of the medium directly influences the determined pressure value negatively.

Moreover, further problems arise when utilizing known pressure sensors in an aggressive medium, which reacts with the sensor's material and thus leads to deterioration, for example, of the membrane or a housing of the sensor. This may result in malfunctioning pressure sensors or unreliable pressure values.

It may therefore be considered as a problem underlying the present invention to provide an improved pressure sensor for pressure measurement in an aggressive medium that avoids at least in part the difficulties encountered in the prior art.

The above problem is at least partly solved by a pressure sensor for pressure measurement in an aggressive medium. It may comprise at least two stationary electrodes and a movable element adapted to move at least in part based on an external pressure. The movable element may be arranged in a vicinity of the at least two electrodes such that an electric field generated by the at least two electrodes changes at least in part based on a movement of the movable element. Such a pressure sensor may allow for a more precise and temporally stable pressure measurement in an aggressive medium so that the above outlined disadvantages of the prior art may at least partly be overcome.

By providing a pressure sensor with at least two stationary electrodes and a movable element in the vicinity of the at least two electrodes, the problems of the prior art can be addressed or resolved individually. One key idea behind the concept of the pressure sensor according to the present disclosure can be understood as a decoupling of any pressure induced (i.e., mechanical) movement from a stationary electrical circuitry measuring a required information/quantity about the pressure induced movement. In other words, the mechanically moving parts and the actively measuring parts may be separated (e.g. the electric field generated by the at least two electrodes may change without the electrodes themselves moving). With respect to actively measuring, the at least two electrodes may be referred to as an active part of the pressure sensor, while the movable element may be referred to as a passive part, i.e., passively influencing the measured quantity of the active part. By this, the movable element may not be electrically connected to the active part of the pressure sensor. This can result in a more stable and precise pressure determination.

For example, the configuration and arrangement of the at least two (stationary) electrodes can be optimized independently from any mechanical requirements since a movement of these electrodes is neither foreseen nor needed. Also, since the electrodes are not foreseen to be movable, they do not need to be adapted to be compliant with the surroundings of the sensor that typically induces movement of the movable element of a pressure sensor. Instead, the movable element may be particularly tailored for compatibility with the surroundings of the sensor. Hence, materials that are particularly stable even in aggressive media may be used. On the other hand, the movable element can be individually optimized for a high-precision and improved movement induced by an external pressure (change) without a drawback of disturbing the arrangement of the at least two electrodes negatively.

In some embodiments, the pressure sensor may be an implantable pressure sensor. The implantable pressure sensor may be adapted for being inserted into a human or animal body, particularly into a blood circulation for determining a blood pressure, for example in a blood vessel, in particular the pulmonary artery, and/or heart. In this embodiment, the aggressive medium may be blood.

Additionally or alternatively, all surfaces of the pressure sensor which are in direct contact with the aggressive medium may be biocompatible and/or bio-stable. In this manner, a degradation of the pressure sensor over time based on its surroundings can be avoided. Further, an unintended and dangerous immune response or reaction of a patient's body can be prevented or at least reduced to a great extent.

The at least two electrodes may be arranged in a plane. Contrary to a standard capacitor arrangement, in which the largest surfaces of the electrodes are generally opposing each other, the electrode arrangement according to the present invention may be understood as arranging the at least two electrodes in the same plane next to each other. Accordingly, the ratio of the electric field expanding out of the plane in which the at least two electrodes are arranged compared to emerging in said plane may be increased with respect to a standard capacitor arrangement. By this, the change of electric field based on the movement of the movable element in the vicinity of the at least two electrodes may be enhanced.

For example, the at least two electrodes may comprise at least two (planar) finger-shaped elements that are arranged adjacent to each other. For example, a first electrode may comprise a first finger-shaped element, and a second electrode may comprise a second finger-shaped element, wherein the first and second finger-shaped elements are arranged adjacent to each other. For example, they may be arranged adjacent to each other such that long sides of the finger-shaped elements face each other and are essentially parallel to each other. For example, the finger-shaped elements may comprise a similar and/or the same width and length, and both may be arranged such that a gap between the long sides may be about 0.1 to 10 times or 0.5 to 5 times of the (average) width of both finger-shaped elements.

The at least two electrodes may be interdigitated electrodes, IDE. A first electrode may comprise a plurality of finger-shaped elements (in the following: "fingers"). A second electrode may comprise at least one finger that is arranged in between two of the fingers of the first electrode, wherein the fingers may be arranged in a plane and essentially parallel to each other. It is also possible that each interdigitated electrode may comprise multiple fingers. By increasing the number of fingers of each electrode, the in-plane extent of the two electrodes can be increased. This may result in an enlarged and/or more homogeneous out of plane electric field. Based thereon, the changes of the electric field based on the movement of the movable element can be determined more accurately leading to a more accurate and precise pressure measurement.

A surface of the movable element facing the at least two electrodes may be electrically conductive or may comprise a dielectric. In either case, since said surface of the movable element interacts with the electric field generated by the at least two electrodes, changing the distance of said surface to the at least two electrodes influences a behavior of the at least two electrodes when a voltage is applied. For example, when an AC (alternating current) voltage is applied to the at least two electrodes, a (combined) impedance of the at least two electrodes may depend on the distance of the at least two electrodes to said surface of the movable element. Thus, since a movement of the movable element is at least partly based on the external pressure, the (combined) impedance of the at least two electrodes may depend on the external pressure. It may be noted that other quantities of the at least two electrodes and/or applying a DC (direct current) voltage may also be applicable for pressure measurements according to the present invention. A change of the electric field generated by the at least two electrodes may be increased, if said surface of the movable element is electrically conductive. Such embodiments may be preferred. In case the movable element comprises a material that is electrically insulating/non-conductive, a deposition of a small metallization layer on said surface of the movable element may be applicable. Said surface of the movable element may be the closest surface of the movable element with respect to the at least two electrodes. In case the movable element and/or its surface does not comprise a conductive material, it may be preferably to use a dielectric with a relatively high permittivity, e.g. at least 1.4, at least 2, or at least 5.

The movable element may be arranged essentially parallel to the at least two electrodes. In this manner, the electric field generated by the at least two electrodes may be changed or influenced by the movable element in a homogenous manner across the at least two electrodes. Thus, inhomogeneities in the electric field may be reduced. By this, a precision of the pressure measurement may be enhanced. A linearity of the pressure sensor may also be increased. Additionally, an evaluation algorithm or routine of the pressure based on a measured information may be facilitated.

In some embodiments, the movable element may comprise a membrane and/or a tappet arranged on a membrane. An individual manufacture of the membrane and the tappet may be provided. Further, utilizing different materials for manufacturing the membrane and the tappet may be applicable. Thus, each material may be specifically selected and/or optimized for its intended application. Alternatively, membrane and movable element may be manufactured integrally (e.g. monolithically), saving an additional and/or elaborate attachment step of the movable element onto the membrane. Further, an accuracy of the movable element may be improved by embodying the membrane and/or the tappet integrally.

A maximum displacement of the movable element based on an excess pressure may be set when the movable element physically contacts the at least two electrodes. By this, a bursting of the membrane may be prevented.

The pressure sensor may further comprise a base element. The at least two electrodes may be arranged on the base element, e.g. a base plate. The base element may comprise or may essentially consist of titanium, stainless steel, glass, or ceramic. If the base element comprises a conductive material, a (thin) insulating layer may be arranged between the based plate and the at least two electrodes. The insulating layer may be adapted to electrically decouple the at least two electrodes from the base plate. The possibility of utilizing various materials for the base plate may enable to specifically select the material based on an intended surrounding and/or aggressive medium of the pressure sensor. Further, the base plate may be arranged on an additional part of a device for measuring a pressure, for example a housing of the device (e.g., including a battery, a wireless transmitter or transceiver, etc.), or arranged next to an additional (similar or different) sensor. In this manner, the material of the base plate may be adapted to the material of the additional part or sensor.

The pressure sensor may further comprise a lid, wherein the lid may comprise the movable element. The lid and the movable element may be manufactured integrally. The lid and the base element may enclose the at least two electrodes at least partially. In some embodiments, the lid and the base element may create a cavity. The cavity may be hermetically sealed comprising a known reference pressure, preferably vacuum, and may enclose the at least two electrodes. Thus, the at least two electrodes may be protected/shielded by the cavity from getting in contact with the aggressive medium and/or electric stray fields. By this, the material of the at least two electrodes can be solely selected based on its sensing properties without requiring any degree of resistance against the aggressive medium, and/or noise may be reduced. Alternatively, the base plate may comprise an opening. The opening may provide a connection between an inner volume of the cavity and a second volume outside the cavity. In this case, the pressure of the second volume may be identical to the pressure inside the cavity, but potentially different to the (external) pressure of the aggressive medium. Thus, the pressure sensor of the present invention may be adapted to measure a relative pressure between two separated volumes/media. The lid and the base element may be attached to each other by welding, gluing, bonding, soldering or similar methods known in the art, wherein welding may be preferred.

The lid may comprise or essentially consist of titanium, stainless steel, glass, or ceramic. The potential of utilizing various materials for the lid may enable to specifically select the material based on an intended surrounding of the pressure sensor. The lid and the base element may comprise the same material. Alternatively, the lid and the base element may comprise different, e.g. individually optimized materials.

In some embodiments, all surfaces of the lid and/or the base element, which are in direct contact to the aggressive medium, may be biocompatible and/or bio-stable. In this manner, a degradation of the pressure sensor over time due to its surroundings, e.g. blood, can be avoided, or at least reduced. Further, an unintended immune response or reaction of a patient's body, in which the pressure sensor according to the present invention has been implanted, can be prevented, or at least reduced by a great extent.

The pressure sensor may further comprise means for determining a (combined) impedance between the at least two electrodes.. In the pressure sensor, the impedance of the at least two electrodes may depend on a movement of the movable element which changes the electric field generated by the at least two electrodes. Since the movement of the movable element is based on the external pressure (change), the determined impedance depends on the external pressure.

The means for determining the impedance may be included in an application-specific integrated circuit, ASIC. Generally, an ASIC corresponds to a small integrated circuit customized for a particular use, rather than intended for general-purpose use. ASICs in mass production provide a cost efficient and reliable integrated circuit. Further, the at least two electrodes may be arranged on the ASIC and/or arranged next to the ASIC. In this manner, a size of the sensor may be reduced.

The pressure sensor may further comprise means to wirelessly transmit the determined impedance, or any information based on the determined impedance (e.g. as a raw signal or a calibrated pressure signal, etc.) as analogue or digital signal. This may be particularly relevant if the pressure sensor cannot be directly wired based on its location, for example when being implanted into a human or animal body. The pressure sensor may further comprise or may be connected to powering means, e.g. a battery.

A height of the pressure sensor may be between 0.5 and 5.0 mm, preferably between 1.5 and 4.0 mm, and more preferably 3 mm. A length of the pressure sensor may be between 1 and 10 mm, preferably between 2 and 7 mm, and more preferably 3 mm. In this manner, the pressure sensor may be suitable to be implanted into a human or animal body to, for example, measuring a blood pressure.

Aspects of the present invention are described in more detail in the following by reference to the accompanying Figures. These Figures show:
- Fig. 1: an illustration of a cross sectional view of an embodiment of a pressure sensor according to the present invention;
- Fig. 2: an illustration of an embodiment of interdigitated electrodes in a top view.
In the following, exemplary embodiments of the present invention are described in more detail, with reference to a pressure sensor. While specific feature combinations are described in the following with respect to the exemplary embodiments of the present invention, it is to be understood that the disclosure is not limited to such embodiments. In particular, not all features have to be present for realizing the invention, and the embodiments may be modified by combining certain features of one embodiment with one or more features of another embodiment.

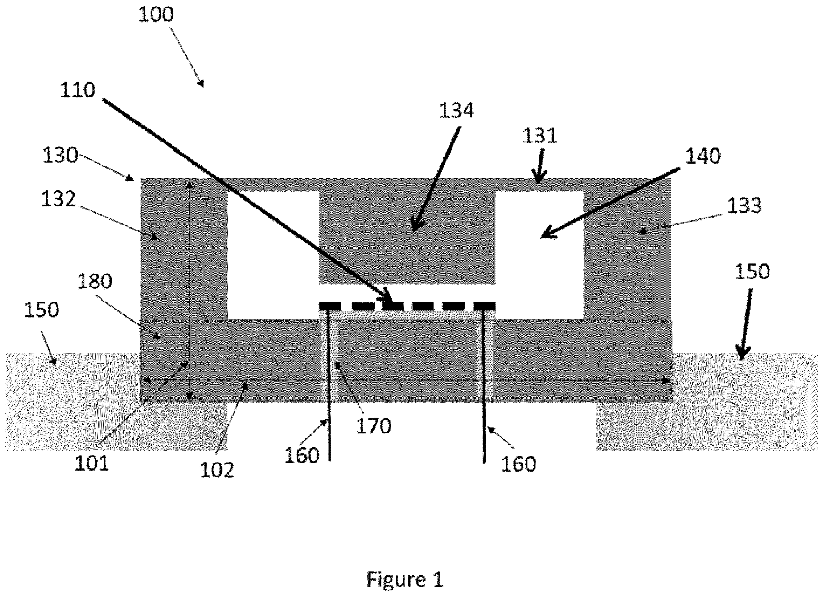

Fig. 1 depicts a cross section of an embodiment of a pressure sensor 100 for pressure measurement in an aggressive medium according to the present invention. The pressure sensor 100 comprises two interdigitated electrodes (IDE) 110, each having three fingers. It may be understood that the present invention may not be limited to IDE's having three fingers. Instead, different quantities of fingers are also applicable, e.g. each electrode may have one, two, three or more than three fingers, for example. The two electrodes are stationary and arranged on a base plate 180, wherein a thin layer of insulating material electrically decouples the two electrodes from the base plate. In case the base plate comprises electrically insulating material, an additionally insulating layer may be omitted. The base plate may comprise or may essentially consist of titanium, stainless steel, glass, or ceramic, wherein titanium is generally preferred. Each electrode 110 is contacted by a conductive wire 160. Each conductive wire 160 is enclosed by an insulating feed-trough 170. The feed-through 170 may be pressure tight.

The pressure sensor 100 further comprises a lid 130. The lid 130 is integrally formed and includes a membrane 131, an all-round side wall (left sidewall 132 and right sidewall 133 shown in Fig. 1) and a tappet 134. The lid 130 may comprise or may essentially consist of titanium, stainless steel, glass, or ceramic, preferably titanium. An individual manufacturing process combined with an additional attachment process of some or even all parts 131-134 of the lid 130 may also be applicable.

The lid 130 together with the base plate 180 may form a cavity 140 enclosing the two electrodes 110. The cavity 140 may be hermetically sealed including a known reference pressure, preferably vacuum. Other pressure values, e.g., atmospheric pressure or excess pressure, may also be applicable and selected based on an intended application of pressure sensor 100.

The membrane 131 may be in contact with the aggressive medium. Further, the membrane 131 is adapted as a moveable element and to move at least in part based on an external pressure (change) originating from the aggressive medium. A thickness of the membrane 131 may preferably be selected from the range from 2 µm to 100 µm, 5 to 50 µm, or 15 µm to 25 µm, while other thicknesses are also applicable. As can be seen from Fig. 1, tappet 134 and membrane 131 are integrally formed such that the tappet 134 is directly arranged above the two electrodes 110. In this manner, a movement of the membrane caused by the pressure change simultaneously moves the tappet 134 closer or further away from the two electrodes 110. The lower surface of the tappet 134 closest to the two electrodes 110 is essentially parallel to the two electrodes 110.

An AC voltage (not shown), e.g. powered by a battery, may be applied to the two electrodes (110).. Based thereon an oscillating electric field emerges between and most importantly above the two electrodes 110. The oscillations may be based on reversing a polarity of the electric field due to the AC voltage. Based on the oscillating electric field, a (combined) impedance of the two electrodes 110, in particular a capacitive portion of the impedance, can be measured via the conductive wires 160 and/or determined by an ASIC (not shown), or a similar integrated circuit connected to the conductive wires 160. Any conductive or dielectric medium within a vicinity of the two electrodes influences the oscillating electric field. Based on that influence, a change in impedance can be determined. Since the movement of the tappet 134 is at least partly based on the external pressure, the change in impedance can be translated into a change in determined pressure or an absolute determined pressure value. In this manner, a characteristic of the measured quantity may only depend on the properties of the membrane 131. It may be noted that the membrane 131 may be adapted to bend in both directions (e.g. by selecting a suitable pressure inside cavity 140), i.e., bringing the tappet 134 closer to the two electrodes 110, when the external pressure increases, or pulling the tappet 134 further away from the two electrodes 110, when the external pressure decreases. In other words, pressure sensor 100 may adapted to determine both, positive and negative pressure changes.

The pressure sensor 100 may further comprise a transmitting unit and an antenna (not shown) for wirelessly transmitting the determined impedance, or any information based on the determined impedance as analogue or digital signal. The pressure sensor 100 may further comprise a receiving unit (not shown), which is adapted to receive wirelessly transmitted signals. Receiver and transmitter may form a transceiver. The signals may include information required by the pressure sensor 100, such as calibration information or similar.

A height 101 of the pressure sensor 100 may be between 0.5 and 5.0 mm, preferably 3 mm. A length 102 of the pressure sensor 100 may be between 1 and 10 mm, preferably 3 mm. In this manner, the pressure sensor may be suitable to be implanted into a human or animal body to, for example, measure a blood pressure. The pressure sensor may have a generally round or rectangular shape and/or may comprise rounded edges.

As can be seen from Fig. 1, the pressure sensor 100 is arranged on a further plate 150. Plate 150 may correspond to a housing of the pressure sensor device including the pressure sensor 100. Further, in case the pressure sensor 100 does not include any powering means (e.g. battery) or further means (e.g. transceiver), these may be arranged inside the pressure sensor device connected to the pressure sensor 100. The pressure sensor device may also comprise means for anchoring it to tissue of a blood vessel or a heart, for example.

Most or even all outer surfaces of pressure sensor 100 may comprise material, which is resistant against the aggressive medium the pressure sensor 100 is intended to be used in and/or surrounded with. In particular, if pressure sensor 100 is implanted into a human or animal body, the material of the outer surfaces of pressure sensor 100 may be bio-compatible and/or bio-stable.

It may be noted that pressure sensor 100 may be fully functioning without requiring a coupling liquid inside the cavity or strain transducers attached to the membrane. Hence, pressure sensor 100 may be free from coupling liquid and no strain transducers may be attached to the membrane. Further, membrane 131 may not be required to be in electrical contact with any internal or external defined electric potential. In particular, membrane 131 can be electrically floating, and it may not be in electrical contact with the electrodes and/or the circuitry reading out the electrodes. Moreover, most, or even all parts of pressure sensor 100 may be manufactured using a well-established micro-electromechanical system (MEMS) technology having low manufacturing uncertainties while being cost-efficient.

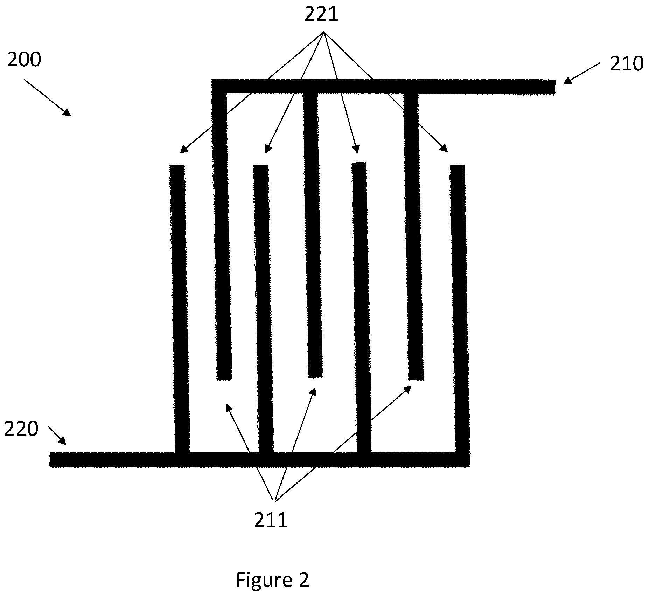

Fig. 2 depicts an embodiment of an IDE 200 in a top view. IDE 200 comprises two electrodes 210, 220, which are arranged in a plane. Electrode 210 comprises three fingers 211 and electrode 220 comprises four fingers 221. In this manner, fingers 221 of electrode 220 are surrounding fingers 211 of electrode 210 in the plane of the IDE 200, which may result in a more symmetric electrical field generated by the two electrodes 210, 220. While various numbers of fingers may be applicable, it may be foreseen that a first electrode has a number of n fingers (n = 1, 2, ...) and a second electrode may have the same number of fingers or it may have n+1 fingers. By increasing the number of fingers, the in-plane extent of the electrical field may be increased. Based thereon, the change of electric field based on the influence of the movable element may be determined more accurately.

## Claims

1. A pressure sensor (100) for pressure measurement in an aggressive medium comprising:
at least two stationary electrodes (110, 210, 220);
a movable element (131, 134) adapted to move at least in part based on an external pressure;
wherein the movable element (131, 134) is arranged in a vicinity of the at least two electrodes (110, 210, 220) such that an electric field generated by the at least two electrodes (110, 210, 220) changes at least in part based on a movement of the movable element (131, 134).

2. The pressure sensor (100) according to claim 1, wherein the pressure sensor (100) is an implantable pressure sensor, which implantable pressure sensor is in particular adapted for being inserted into a human or animal blood circulation.

3. The pressure sensor (100) according to claim 1 or 2, wherein all surfaces of the pressure sensor (100) which are in direct contact to the aggressive medium are biocompatible and/or bio-stable.

4. The pressure sensor (100) according to any of the previous claims, wherein the at least two electrodes (110, 210, 220) are arranged in a plane.

5. The pressure sensor (100) according to any of the previous claims, wherein the at least two electrodes (110, 210, 220) are interdigitated electrodes (110, 210, 220), IDE.

6. The pressure sensor (100) according to any one of the previous claims, wherein a surface of the movable element (131, 134) facing the at least two electrodes (110, 210, 220) is electrically conductive or comprises a dielectric.

7. The pressure sensor (100) according to any one of the previous claims, wherein the movable element (131, 134) is arranged essentially parallel to the at least two electrodes (110, 210, 220).

8. The pressure sensor (100) according to any of the previous claims, wherein the movable element (131, 134) comprises a membrane (131) and/or a tappet (134) arranged on a membrane (131).

9. The pressure sensor (100) according to any one of the previous claims, further comprising a base element (180), wherein the at least two electrodes (110, 210, 220) are arranged on the base element (180).

10. The pressure sensor (100) according to the previous claim, wherein the base element (180) comprises or essentially consists of titanium, stainless steel, glass, or ceramic.

11. The pressure sensor (100) according to claim 9 or 10, further comprising a lid (130), wherein the lid (130) comprises the movable element (131, 134); and
wherein the lid (130) and the base element (180) enclose the at least two electrodes (110, 210, 220) at least partially.

12. The pressure sensor (100) according to the previous claim, wherein the lid (130) comprises or essentially consists of titanium, stainless steel, glass, or ceramic.

13. The pressure sensor (100) according to any one of the previous claims, further comprising means for determining an impedance between at least two electrodes (110, 210, 220).

14. The pressure sensor (100) according to the previous claim, wherein the means for determining the impedance are included in an application-specific integrated circuit, ASIC, and the at least two electrodes (110, 210, 220) are arranged on the ASIC and/or arranged next to the ASIC.

15. The pressure sensor (100) according to claim 13 or 14, wherein the pressure sensor (100) further comprises means to wirelessly transmit the determined impedance, or any information based on the determined impedance as analogue or digital signal.
